# EUROPEAN PATENT SPECIFICATION

(11) **EP 3 527 215 B1**
(45) Date of publication and mention of the grant of the patent: **21.04.2021**
(21) Application number: 19158239.4
(22) Date of filing: 20.02.2019
(51) Int. Cl.: A61K 35/36, A61K 9/00, C12Q 1/48, A61K 35/76

(54) **A METHOD USING AN AGENT FOR PROMOTING EXPRESSION OF N-ACETYLGALACTOSAMINYLTRANSFERASE CONTAINING EXTRACT FROM INFLAMED TISSUES INOCULATED WITH VACCINIA VIRUS**
VERFAHREN MIT VERWENDUNG EINES MITTELS ZUR FÖRDERUNG DER EXPRESSION VON N-ACETYLGALACTOSAMINYLTRANSFERASE-HALTIGEM EXTRAKT AUS MIT VACCINIAVIRUS INOKULIERTEN ENTZÜNDETEN GEWEBEN
PROCÉDÉ UTILISANT UN AGENT POUR PROMOUVOIR L'EXPRESSION DE N-ACÉTYLGALACTOSAMINYLTRANSFÉRASE CONTENANT UN EXTRAIT PROVENANT DE TISSUS ENFLAMMÉS INOCULÉS AVEC LE VIRUS DE LA VACCINE

(30) Priority: 20.02.2018 JP 2018027931
(43) Date of publication of application: 21.08.2019
(73) Proprietor: TOKAI UNIVERSITY EDUCATIONAL SYSTEM, Tokyo 151-0063 (JP); NIPPON ZOKI PHARMACEUTICAL CO., LTD., Chuo-ku Osaka-shi Osaka 541-0046 (JP)
(72) Inventor: Sakai, Daisuke, Kanagawa 2591193 (JP); Nakai, Tomoko, Kanagawa 2591193 (JP); Naiki, Mitsuru, Hyogo 6731461 (JP); Shibayama, Yoji, Hyogo 6731461 (JP)
(74) Representative: Hoffmann Eitle

(56) References cited:
- WO-A2-2012/051173
- OSHIDA TATSUHIRO ET AL: "Effect of a nonprotein bioactive agent on the reduction of cyclooxygenase-2 and tumor necrosis factor-alpha in human intervertebral disc cells in vitro", JOURNAL OF NEUROSURGERY. S, AMERICAN ASSOCIATION OF NEUROLOGICAL SURGEONS, US, vol. 9, no. 5, 1 November 2008 (2008-11-01), pages 411-418, XP008164512, ISSN: 1547-5654, DOI: 10.3171/SPI.2008.9.11.411
- DAISUKE SAKAI ET AL: "Upregulation of glycosaminoglycan synthesis by Neurotropin in nucleus pulposus cells via stimulation of chondroitin sulfate N-acetylgalactosaminyltransferase 1: A new approach to attenuation of intervertebral disc degeneration", PLOS ONE, vol. 13, no. 8, 27 August 2018 (2018-08-27), page e0202640, XP055583661, DOI: 10.1371/journal.pone.0202640

## Description

### Technical Field

The present invention relates to novel method for determining or evaluating by an index the pharmacological activity of an extract from inflamed tissues inoculated with vaccinia virus (which will hereinafter sometimes be called the "present extract"). More specifically, the present invention relates to the index being promoting the expression of N-acetylgalactosaminyltransferase (GalNAcT).

### Background Art

An intervertebral disc is composed of three types of tissues, i.e., nucleus pulposus, annulus fibrosus, and cartilaginous endplates, that biochemically and physically differ in their characteristics. About 80% of an intervertebral disc is water because rich proteoglycan in the nucleus pulposus retains plenty of water. Intervertebral disc degeneration is one of the main causes of lower back pain and is also associated with the development of diseases such as spinal disc herniation and spinal stenosis. Although age-related intervertebral disc degeneration varies widely from person to person, changes in cells that constitute the nucleus pulposus part, thus-caused changes in the matrix, and subsequent breakdown of the structure of the annulus fibrosus are considered to be closely associated with intervertebral disc degeneration. At the age of 60 and older in humans, the proteoglycan content in intervertebral discs decreases to from 65% to less than a half (about 30%) of that of teenagers. As a result, the water content in intervertebral discs decreases. This condition makes intervertebral discs more prone to structure breakdown triggered by external factors.

Proteoglycans are a compound composed of multiple polysaccharide chains (glycosaminoglycan or GAG), which are each formed from repeated disaccharide units, such as chondroitin sulfate, and the polysaccharide chains are each bound to a tetrasaccharide binding domain formed of sugars binding to a serine residue of a core protein. Proteoglycans have diverse functions as a biogenic substance and are present in various major organs, the brain, the skin, and the extracellular matrix and cell surfaces of tissues throughout the body, as well as being the main component of cartilage.

Glycosaminoglycans are classified into four types according to the difference in the backbone of their disaccharides: (1) heparin, heparan sulfate, (2) chondroitin sulfate, dermatan sulfate, (3) keratan sulfate, and (4) hyaluronic acid. These backbones are subject to modification in various ways, typically sulfation. In most cases, GAG is bound to a core protein; i.e., GAG is present in the form of a proteoglycan. Biosynthesis of GAG is known to involve various glycosyltransferases and sulfotransferases.

Chondroitin sulfate is a type of GAG and is abundant as a side chain of aggrecan, which is the major component of the extracellular matrix of cartilage. Chondroitin sulfate is known to be decreased in cartilage of patients with intervertebral disc degeneration disease or osteoarthritis (OA).

Chondroitin sulfate is a linear sulfated sugar chain composed of disaccharides that alternately repeat N-acetylgalactosamine (GalNAc) and glucuronic acid (GlcUA). The biosynthesis of the sugar chain skeleton of chondroitin sulfate involves two types of N-acetylgalactosaminyltransferases: one is N-acetylgalactosaminyltransferase 1 (GalNAcT1) that transfers GalNAc to the GlcUA residue at the terminus of the binding domain, and the other is N-acetylgalactosaminyltransferase 2 (GalNAcT2) that transfers GalNAc to the GlcUA terminus of the repeated disaccharides. The biosynthesis of chondroitin sulfate begins with the transfer of GalNAc to the GlcUA residue at the terminus of the binding domain by GalNAcT1. Thereafter, GlcUA is transferred by glucuronosyltransferase, and then GalNAc is transferred by GalNAcT2. These transfers are repeated alternately to synthesize the repeated unit domain of the disaccharides, thereby extending the sugar chains. Finally, the sugars are sulfated by sulfotransferase to generate chondroitin sulfate. Thus, it has become clear that in the biosynthesis of chondroitin sulfate, GalNAcT1 is involved in starting the synthesis of a sugar chain, and that GalNAcT2 extends the sugar chain. In other words, GalNAcT1 is involved in not extending the sugar chain of chondroitin sulfate, but in increasing the number of sugar chains.

The extract from inflamed tissues inoculated with vaccinia virus (the present extract) that is contained in the agent for promoting the expression of N-acetylgalactosaminyltransferase according to the present invention or a preparation containing the extract is known to have enormously wide-ranging actions and effects, such as analgesic action, sedative action, antistress action, anti-allergic action, immunostimulating action, anticancer action, cirrhosis inhibition activity, therapeutic effects on idiopathic thrombocytopenic purpura, therapeutic effects on postherpetic neuralgia, brain edema, dementia, spinocerebellar degeneration etc., therapeutic effects on Raynaud syndrome, diabetic neuropathy, SMON aftereffects etc., kallikrein production inhibition action, peripheral circulatory disorder improvement action, bone atrophy improvement action, nitrogen monoxide production inhibition action effective in treating sepsis or endotoxin shock, therapeutic effects on osteoporosis, therapeutic effects on AIDS based on Nef action inhibition activity or chemokine production inhibition activity, therapeutic effects on ischemic diseases such as cerebral infarction, therapeutic effects on fibromyalgia, therapeutic effects on infection, preventive or alleviating action on peripheral nerve disorder caused by anticancer agents, therapeutic effects on chronic prostatitis, interstitial cystitis and/or urination disorder, neurotrophic factor (e.g., BDNF) production promotion action, protective action on the liver, migration enhancement action on pluripotent stem cells (Muse cells), and preventive or therapeutic effects on muscle damage. In addition, the present extract or a preparation containing the extract is known to have promoting action on the synthesis of collagen and proteoglycan in cartilage cells (see PTL 1). However, it remains unknown that the present extract or a preparation containing the extract promotes the expression of GalNAcT, and that the present extract or a preparation containing the extract promotes the expression of GalNAcT1 more than GalNAcT2. Promoting the expression of GalNAcT1 more than GalNAcT2 indicates that the action to increase the number of sugar chains is greater than the action to extend the sugar chain of chondroitin sulfate. Medicinal agents having such action have been unknown.

### Citation List

### Patent Literature

PTL 1: WO2012/051173

### Summary of Invention

### Technical Problem

The present invention provides an agent for promoting the expression of GalNAcT containing the present extract.

### Solution to Problem

The present inventors conducted extensive research on pharmacological action of the present extract and found that the present extract has excellent GalNAcT expression promotion action. The inventors then completed the present invention.

### Advantageous Effects of Invention

Because the present extract has GalNAcT expression promotion action, a preparation containing the present extract can be an excellent therapeutic or preventive preparation for diseases associated with intervertebral disc degeneration and osteoarthritis. In particular, because preparations containing the present extract have long been used as a highly safe medicinal agent with fewer drawbacks such as side effects, the present invention is very useful.

### Description of Embodiments

The present extract is an extract containing a non-proteinous active substance extracted and separated from the inflamed skin tissues of animals by the inoculation of vaccinia virus. Although the present extract is liquid in an extracted state, it is also possible to make into a solid by means of drying. The present preparation is very useful as a drug. In a specific product as the present preparation which is manufactured and distributed by the applicant, there is "a preparation containing an extract from inflamed skins of rabbits inoculated with vaccinia virus" (trade name: NEUROTROPIN [registered trademark]; hereinafter, it will be referred to as "NEUROTROPIN"). In NEUROTROPIN, there are injection and tablet and both belong to an ethical drug.

Indications of NEUROTROPIN injection are "low back pain, cervicobrachial syndrome, symptomatic neuralgia, itchiness accompanied by skin diseases (eczema, dermatitis, urticaria), allergic rhinitis and sequelae of subacute myelo-optico-neuropathy (SMON) such as coldness, paresthesia and pain". Indications of NEUROTROPIN tablet are "postherpetic neuralgia, low back pain, cervicobrachial syndrome, periarthritis scapulohumeralis and osteoarthritis". NEUROTROPIN preparation has been created and developed as a drug by the applicant. NEUROTROPIN preparation has been appreciated for its excellent advantage for efficacy and safety, sold for many years and established a firm position in the Japanese pharmaceutical market.

The extract from inflamed tissues inoculated with vaccinia virus used in the present invention can be obtained by the following manner: inflamed tissues inflamed by the inoculation with vaccinia virus is crushed; an extraction solvent is added to remove the tissue fragments; then deproteinization is carried out; the deproteinized solution is adsorbed onto an adsorbent; and then the active ingredient is eluted, for example, according to the following process.
(A) Inflamed skin tissues of rabbits, mice or the like by the inoculation with vaccinia virus are collected, and the inflamed tissues are crushed. To the crushed tissue an extraction solvent such as water, phenolated water, physiological saline or phenol-added glycerin water is added. Then, the mixture is filtered or centrifuged to obtain an extraction liquid (filtrate or supernatant).
(B) The pH of the extraction liquid is adjusted to be acidic and the liquid is heated for deproteinization. Then, the deproteinized solution is adjusted to be alkaline, heated, and then filtered or centrifuged.
(C) The obtained filtrate or supernatant is made acidic and adsorbed onto an adsorbent such as activated carbon or kaolin.
(D) To the adsorbent, an extraction solvent such as water is added, the pH is adjusted to alkaline, and the adsorbed component is eluted to obtain the extract from inflamed tissues inoculated with vaccinia virus. Subsequently, as desired, the eluate may be evaporated to dryness under reduced pressure or freeze-dried to give a dried material.

As for animals in order to obtain the inflamed tissues by the inoculation of vaccinia virus, various animals that is infected with vaccinia virus such as rabbits, cows, horses, sheep, goats, monkeys, rats or mice can be used, and preferred inflamed tissues are inflamed skin tissues of rabbits. With regard to a rabbit, any rabbit may be used so far as it belongs to Lagomorpha. Examples thereof include Oryctolagus cuniculus, domestic rabbit (domesticated Oryctolagus cuniculus), hare (Japanese hare), mouse hare and snowshoe hare. Among them, it is appropriate to use domestic rabbit. In Japan, there is family rabbit called "Kato" which has been bred since old time and frequently used as livestock or experimental animal and it is another name of domestic rabbit. There are many breeds in domestic rabbit and the breeds being called Japanese white and New Zealand white are advantageously used.

Vaccinia virus used herein may be in any strain. Examples thereof include Lister strain, Dairen strain, Ikeda strain, EM-63 strain and New York City Board of Health strain.

As to basic extracting steps (A) to (D) of the above-described for the present extract can be carried out in more detail, the following steps are used for example. About step (A):
The inflamed skin tissues of rabbits by the intradermal inoculation of vaccinia virus are collected. The collected skin tissues are washed and disinfected using a phenol solution, etc. This inflamed skin tissues are crushed and an extraction solvent in 1- to 5-fold thereof by volume is added thereto. Here, the term "crush" means to finely break down into minces using a mincing machine or the like. As to the extraction solvent, there may be used distilled water, physiological saline, weakly acidic to weakly basic buffer, etc. and bactericidal/antiseptic agent such as phenol, stabilizer such as glycerin, salts such as sodium chloride, potassium chloride or magnesium chloride, etc. may be appropriately added thereto. At that time, it is also possible that the cell tissue is destroyed by a treatment such as freezing/melting, ultrasonic wave, cell membrane dissolving enzyme or surfactant so as to make the extraction easier. The resulting suspension is allowed to stand for 5 to 12 days. During that period, the suspension may be heated at 30 to 45°C with or without appropriate stirring. The resulting liquid is subjected to a treatment for separating into solid and liquid (filtered or centrifuged, etc.) to remove the tissue fragments whereupon a crude extract (filtrate or supernatant) is obtained.

### About step (B)

The crude extract obtained in step (A) is subjected to a deproteinizing treatment. The deproteinization may be carried out by a known method which has been usually conducted and a method such as heating treatment, treatment with a protein denaturant (such as acid, base, urea, guanidine or an organic solvent including acetone), isoelectric precipitation or salting-out may be applied. After that, a common method for the removal of insoluble matters such as filtration using filter paper (such as cellulose or nitrocellulose), glass filter, Celite or Seitz filter, ultrafiltration or centrifugation is conducted to give a filtrate or a supernatant wherefrom the separated insoluble protein is removed.

### About step (C)

The filtrate or supernatant obtained in step (B) is adjusted to acidic or, preferably, to pH 3.5 to 5.5 to conduct an operation of adsorbing with an adsorbent. Examples of the usable adsorbent include activated carbon and kaolin. An adsorbent is added to the extract followed by stirring or the extract is passed through a column filled with an adsorbent so that the active ingredient can be adsorbed with the adsorbent. When an adsorbent is added to the extract, the adsorbent with which the active ingredient is adsorbed can be obtained by means of filtration, centrifugation, etc. to remove the solution.

### About step (D)

For elution (desorption) of the active ingredient from the adsorbent obtained in step (C), an elution solvent is added to said adsorbent and adjusted to basic or, preferably, to pH 9 to 12, elution is conducted at room temperature or with suitable heating, or with stirring, and then the adsorbent is removed by a common method such as filtration or centrifugation. As to the extraction solvent used therefore, there may be used a basic solvent such as water, methanol, ethanol, isopropanol or the like adjusted to basic pH or an appropriate mixed solvent thereof and preferably, water adjusted to pH 9 to 12 may be used. Amount of the extracting solvent may be appropriately set. In order to use the eluate obtained as such as a drug substance, the pH is appropriately adjusted to nearly neutral or the like whereby an extract from inflamed skins of rabbits inoculated with vaccinia virus (the present extract) can be finally obtained.

Since the present extract is liquid at the stage of being prepared, it is also possible that said extract is appropriately concentrated or diluted to make into a desired concentration. When a preparation is manufactured from the present extract, it is preferred to apply a sterilizing treatment with heating. For making into an injectable preparation, it is possible to add sodium chloride or the like so as to prepare a solution being isotonic to physiological saline. It is also possible that the present extract is orally administered in a liquid or gel state. Furthermore, the present extract may be subjected to an appropriate operation such as concentration to dryness to prepare a solid preparation for oral administration such as a tablet. Specific methods for the manufacture of solid preparation for oral administration from the present extract are disclosed in the specifications of Japanese Patent Nos. 3,818,657 and 4,883,798. Examples of the present preparation includes an injectable preparation, a solid preparation for oral administration, etc. prepared as such.

The following describes examples of methods for producing the present extract, as well as clinical evaluation concerning novel pharmacological activity of the extract, and the expression promotion action on N-acetylgalactosaminyltransferase. The present invention is not limited to the descriptions in the Examples , but rather it is defined in the appended claims.

### Examples

### Example 1: Manufacture of the Present Extract

Skins of healthy adult rabbits were inoculated with vaccinia virus intradermally and the inflamed skins were cut and collected. The collected skins were washed and disinfected by a phenol solution, an excessive phenol solution was removed and the residue was crushed. A phenol solution was added thereto and mixed therewith and the mixture was allowed to stand for 3 to 7 days, and further heated at 35 to 40°C together with stirring for 3 to 4 days. After that, an extracted solution obtained by a solid-liquid separation was adjusted to pH 4.5 to 5.2 with hydrochloric acid, heated at 90 to 100°C for 30 minutes and filtered to remove protein. The filtrate was adjusted to pH 9.0 to 9.5 with sodium hydroxide, heated at 90 to 100°C for 15 minutes and subjected to a solid-liquid separation.

The resulting deproteinized solution was adjusted to pH 4.0 to 4.3 with hydrochloric acid, activated carbon in an amount of 2% to the mass of the deproteinized solution was added thereto and the mixture was stirred for 2 hours and subjected to the solid-liquid separation. Water was added to the collected activated carbon followed by adjusting to pH 9.5 to 10 with sodium hydroxide and the mixture was stirred at 60°C for 90 to 100 minutes and centrifuged to give a supernatant. Water was added again to the activated carbon precipitated upon the centrifugation followed by adjusting to pH 10.5 to 11 with sodium hydroxide and the mixture was stirred at 60°C for 90 to 100 minutes and centrifuged to give a supernatant. Both supernatants were combined and neutralized with hydrochloric acid to give the present extract.

### Example 2: Test Method and Test Results

The following describes the method of a pharmacological test conducted for demonstrating the GalNAcT expression promotion action in intervertebral disc cells due to the present extract obtained in Example 1, as well as the test results.

### Cell and Reagent

Test Examples 1 to 3 used human nucleus pulposus cells prepared in accordance with the following procedure approved by the Experimentation Ethics Committee of Tokai University School of Medicine. Nucleus pulposus tissue was harvested from five patients with spinal disc herniation (three males and two females, aged 29 to 38) during surgery with their consent. The nucleus pulposus tissue was cut into fragments and treated with TrypLE Express (Gibco) for 1 hour, followed by treatment with 0.25 mg/ml Collagense-P (Roche) for 2 hours. Cells isolated at 37°C were washed with an α-MEM medium (Wako Chemical) twice and seeded at a density of about 5 × 10³/cm². The cells were cultured in an α-MEM medium containing 10% fetal bovine serum (FBS, Sigma-Aldrich), 100 U/ml penicillin (Gibco), and 100 mg/ml streptomycin (Gibco) under 2% O₂ (low oxygen) and 5% CO₂ at 37°C. The medium was replaced twice a week, and the cells were treated with trypsin (Gibco) before reaching confluence to perform subculture. The cells obtained from the third subculture were used in the Examples.

The nucleus pulposus cells were seeded in a 25-cm² flask at a density of 5000/cm² and cultured in an α-MEM medium containing 10% FBS overnight before the present extract was added. Thereafter, the cells were treated with an α-MEM medium containing the present extract, 10% FBS, and 170 µM of ascorbic acid. While the culture was maintained for 2 weeks, the medium was replaced every other day. Nucleus pulposus cells that reached confluence were used in the following tests.

### Statistical Analysis

Statistical analysis was performed with a repeated measures ANOVA. When the p value was less than 0.05, Bonferroni correction was performed.

### Test Example 1: Effect on GAG Expression

### GAG and DNA Analysis

The cultured cells were washed with Dulbecco's phosphate-buffered saline (DPBS, DS-Pharma) and treated with a buffer solution containing 25 mg/ml papain (Sigma-Aldrich), 8 mg/ml sodium acetate (Wako Chemical), 4 mg/ml ethylenediaminetetraacetic acid (Sigma-Aldrich), and 1.57 mg/ml L-cysteine (Sigma-Aldrich) at 65°C overnight. The sulfated GAG content was calculated by measuring the absorbance at 656 nm using 1,9-dimethylmethylene blue (Biocolor), with chondroitin-6-sulfate (Biocolor) as the standard, with a SPECTRA MAX i3 spectrophotometer (Molecular Devices). The amount of DNA was calculated by a PicoGreen assay (ThermoFisher Scientific, Waltham, MA) with excitation at 480 nm and luminescence at 520 nm, using the same spectrophotometer. The ratio of GAG to the amount of DNA (average value ± standard error of the mean) was calculated. Table 1 illustrates some of the results of this test.

**Table 1**

| | The Ratio of GAG to DNA |
|---|---|
| Control | 4.33 ± 0.38 |
| Ascorbic Acid | 5.88 ± 0.73 |
| Ascorbic Acid Present Extract 0.1 mNU/mL | 7.12 ± 1.20** |
| Ascorbic Acid Present Extract 1.0 mNU/mL | 6.05 ± 0.76 |

| | |
|---|---|
| **: p<0.01 vs Control | |

The value of GAG/DNA increased 1.7 times with the group administered the present extract in an amount of 0.1 mNU/mL, and 1.4 times with the group administered the present extract in an amount of 1.0 mNU/mL, compared to that of the control group, exhibiting significant enhancement of GAG production in the group administered the present extract in an amount of 0.1 mNU/mL (Table 1).

### Test Example 2: Effect on Expression of CSGALNACT1, ANG1, or IGF Gene (Quantitative Real-Time PCR)

After 1 week from the addition of the present extract in an amount of 1.0 mNU/mL, the cells were collected and homogenized in a lysis buffer. Total RNA (tRNA) was then prepared using an SV Total RNA isolation system (Promega). 2 µg of tRNA of each sample was reverse-transcribed into cDNA using a High Capacity RNA-to-cDNA kit (Applied Biosystems). The amount of mRNA of GALNACT1 was calculated by the comparative CT method, using glyceraldehyde-3-phosphate dehydrogenase (GAPDH: trade name, pre-developed TaqMan Assay Reagents (Applied Biosystems)) as the internal standard. The following primer and probe (Applied Biosystems) were used: IGF1 (TaqMan Assay ID: Hs03986524_m1), ANGPT1 (TaqMan Assay ID: Hs00181613_m1), and CSGALNACT1 (TaqMan Assay ID: Hs00218054_m1). With the expression level of CSGALNACT1, ANGPT1, or IGF1 in the control taken as 1, the ratio of each group (average value ± standard error of the mean) was calculated. Tables 2 to 4 illustrate some of the results of this test.

**Table 2**

| | Ratio to Expression Level of CSGALNACT1 mRNA in Control Taken as 1 |
|---|---|
| Control | 1.00 |
| Ascorbic Acid | 2.25 ± 0.55 |
| Ascorbic Acid Present Extract 0.1 mNU/mL | 3.95 ± 1.25** |
| Ascorbic Acid Present Extract 1.0 mNU/mL | 2.53 ± 0.43* |

| | |
|---|---|
| *: *p*<0.05 vs Control, **: p<0.01 vs Control | |

**Table 3**

| | Ratio to Expression Level of ANGPT1 mRNA in Control Taken as 1 |
|---|---|
| Control | 1.00 |
| Ascorbic Acid | 3.80 ± 0.91** |
| Ascorbic Acid Present Extract 0.1 mNU/mL | 6.91 ± 1.80** |
| Ascorbic Acid Present Extract 1.0 mNU/mL | 5.44 ± 1.09** |

| | |
|---|---|
| **: p<0.01 vs Control | |

**Table 4**

| | Ratio to Expression Level of IGF1 mRNA in Control Taken as 1 |
|---|---|
| Control | 1.00 |
| Ascorbic Acid | 8.92 ± 3.30** |
| Ascorbic Acid Present Extract 0.1 mNU/mL | 12.7 ± 3.22** |
| Ascorbic Acid Present Extract 1.0 mNU/mL | 9.83 ± 2.17** |

| | |
|---|---|
| **: p<0.01 vs Control | |

The expression level of CSGALNACT1 was confirmed to have been significantly enhanced by adding the present extract in an amount of 0.1 mNU/mL or 1.0 mNU/mL (Table 2). The expression level of ANGPT1 mRNA and the expression level of IGF1 mRNA were also confirmed to have been significantly enhanced by adding the present extract in an amount of 0.1 mNU/mL or 1.0 mNU/mL (Tables 3 and 4).

### Test Example 3: Effect on Expression of CSGALNACT1 mRNA and CSGALNACT2 mRNA (Microarray Analysis)

Gene expressions in nucleus pulposus cells derived from a patient - nucleus pulposus cells treated with the present extract in an amount of 1.0 mNU/mL and untreated nucleus pulposus cells - were compared using a microarray. The present extract and 170 µM of ascorbic acid were added to the cells. tRNA was prepared in the same manner as in Test Example 2, and Cy3-labeled cRNA was prepared using an Low Input Quick Amp Labeling Kit (Agilent Technology). The obtained Cy3-labeled cRNA was subjected to hybridization using a SurePrint G3 Human GE 8x60K v2 Microarray (Agilent Technology) and a Gene Expression Hybridization Kit (Agilent Technology). Thereafter, analysis was performed with an Agilent DNA microarray scanner (Agilent Technology, G2600D SG13164306) in accordance with the AgilentG3_HiSen_GX_1Color (Agilent Technology) protocol.

The fluorescence intensity of each probe was converted to an expression level using Agilent Feature Extraction 11.5.1.1 digitization conversion software (Agilent Technology). Genes expressed at high levels were detected using Gene Spring ver.13 gene expression analysis software (Agilent Technology). Genes involved in GAG synthesis were selected using the Database for Annotation, Visualization and Integrated Discovery (DAVID) 2017 Tool, and Kyoto Encyclopedia of Genes and Genomes (KEGG) PATHWAY Database. With the expression level of a sample that was cultured for the same time period without the present extract (control) taken as 1, the ratio of the expression level after addition of the present extract was calculated. The average of signals for the same gene emitted from multiple probes in a microarray was calculated and used as one piece of data. Table 5 illustrates some of the results of this test.

**Table 5**

| Gene Name (Enzyme Name) | Ratio to Expression Level of Control Taken as 1 |
|---|---|
| Control | 1.00 |
| CSGALNACT1 (chondroitin sulfate N-acetylgalactosaminyltransferase 1) | 1.54 |
| HS3ST3A1 (heparan sulfate D-glucosaminyl 3-O-sulfotransferase-3A) | 1.45 |
| EXTL1 (heparan sulfate GlcA/GlcNAc transferase like 1) | 1.41 |
| CHSY3 (N-Acetylgalactosaminyltransferase Glucuronyltransferase) | 1.37 |
| GLCE (glucuronic acid epimerase) | 1.25 |
| HS3ST5 (heparan sulfate 3-OST-5) | 1.19 |
| HS3ST3B1 (heparan sulfate D-glucosaminyl 3-O-sulfotransferase-3B) | 1.18 |
| HS6ST1 (heparan sulfate 6-O-sulfotransferase) | 1.16 |
| FUT8 (alpha 1,6-fucosyltransferase 8) | 1.14 |
| HS3ST1 (heparan sulfate D-glucosaminyl 3-O-sulfotransferase) | 1.13 |
| CHST6 (corneal N-acetylglucosamine 6-sulfotransferase) | 1.13 |
| CSGALNACT2 (chondroitin sulfate N-acetylgalactosaminyltransferase 2) | 1.12 |
| CHST13 (chondroitin D-N-acetylgalactosamine-4-O-sulfotransferase 3) | 1.12 |
| CHST12 (chondroitin D-N-acetylgalactosamine-4-O-sulfotransferase 2) | 1.09 |
| XYLT2 (xylosyltransferase 2) | 1.08 |

In most genes involved in GAG synthesis, enhancement of the expression due to the addition of the present extract in an amount of 1.0 mNU/mL was observed (Table 5). The expression of CSGALNACT was enhanced by 1.54 times in CSGALNACT1 and 1.12 times in CSGALNACT2 (Table 5).

### Industrial Applicability

As described above, the present extract has action for promoting the expression of N-acetylgalactosaminyltransferases, in particular action for promoting the expression of N-acetylgalactosaminyltransferase 1 more than the expression of N-acetylgalactosaminyltransferase 2. This indicates that an agent for promoting the expression of an N-acetylgalactosaminyltransferase containing the present extract is useful as a therapeutic or preventive preparation for diseases associated with intervertebral disc degeneration or osteoarthritis.

## Claims

1. An *in vitro* method for determining or evaluating by an index the pharmacological activity of an extract from inflamed tissues inoculated with vaccinia virus or a preparation containing the extract, wherein the action of promoting expression of an N-acetylgalactosaminyltransferase in intervertebral disc cells is used as the index.

2. The method according to claim 1, wherein the N-acetylgalactosaminyltransferase is N-acetylgalactosaminyltransferase 1.

3. The method according to claim 1, wherein the N-acetylgalactosaminyltransferase is N-acetylgalactosaminyltransferase 2.

4. The method according to any one of claims 1 to 3, comprising
comparing expression increase rates between the N-acetylgalactosaminyltransferase 1 and the N-acetylgalactosaminyltransferase 2, and
confirming that the N-acetylgalactosaminyltransferase 1 has a higher expression increase rate.

5. The method according to any one of claims 1 to 4, wherein the inflamed tissues are inflamed skin tissues of rabbits.

6. A method for verifying that the extract from inflamed tissues inoculated with vaccinia virus or the preparation satisfies the quality standard by performing the determination or evaluation of any one of claims 1 to 5.

7. The method according to claim 6, wherein the preparation is an injectable preparation or an oral preparation.

## Patentansprüche

1. *In* vitro-Verfahren zum Bestimmen oder Evaluieren der pharmakologischen Aktivität eines Extrakts aus entzündeten Geweben, inokuliert mit Vacciniavirus, oder eines den Extrakt enthaltenden Präparats durch einen Index, wobei die Wirkung der Förderung der Expression einer N-Acetylgalactosaminyltransferase in Bandscheibenzellen als Index verwendet wird.

2. Verfahren gemäß Anspruch 1, wobei die N-Acetylgalactosaminyltransferase N-Acetylgalactosaminyltransferase 1 ist.

3. Verfahren gemäß Anspruch 1, wobei die N-Acetylgalactosaminyltransferase N-Acetylgalactosaminyltransferase 2 ist.

4. Verfahren gemäß irgendeinem der Ansprüche 1 bis 3, umfassend
Vergleichen der Expressionssteigerungsraten zwischen der N-Acetylgalactosaminyltransferase 1 und der N-Acetylgalactosaminyltransferase 2 und
Bestätigen, dass die N-Acetylgalactosaminyltransferase 1 eine höhere Expressionssteigerungsrate aufweist.

5. Verfahren gemäß irgendeinem der Ansprüche 1 bis 4, wobei die entzündeten Gewebe entzündete Hautgewebe von Kaninchen sind.

6. Verfahren zum Verifizieren, dass der Extrakt aus entzündeten Geweben, inokuliert mit Vacciniavirus, oder das Präparat dem Qualitätsanspruch genügt, indem die Bestimmung oder Evaluierung nach irgendeinem der Ansprüche 1 bis 5 durchgeführt wird.

7. Verfahren gemäß Anspruch 6, wobei das Präparat ein injizierbares Präparat oder ein orales Präparat ist.

## Revendications

1. Procédé *in vitro* destiné à déterminer ou à évaluer par un indice l'activité pharmacologique d'un extrait provenant de tissus enflammés inoculés avec le virus de la vaccine ou une préparation contenant l'extrait, dans lequel l'action consistant à favoriser l'expression d'une N-acétylgalactosaminyltransférase dans des cellules de disques intervertébraux est utilisée comme l'indice.

2. Procédé selon la revendication 1, dans lequel la N-acétylgalactosaminyltransférase est la N-acétylgalactosaminyltransférase 1.

3. Procédé selon la revendication 1, dans lequel la N-acétylgalactosaminyltransférase est la N-acétylgalactosaminyltransférase 2.

4. Procédé selon l'une quelconque des revendications 1 à 3, comprenant
la comparaison des taux d'augmentation d'expression entre la N-acétylgalactosaminyltransférase 1 et la N-acétylgalactosaminyltransférase 2, et
la confirmation que la N-acétylgalactosaminyltransférase 1 présente un taux d'augmentation d'expression plus élevé.

5. Procédé selon l'une quelconque des revendications 1 à 4, dans lequel les tissus enflammés sont des tissus cutanés enflammés de lapins.

6. Procédé destiné à vérifier si l'extrait provenant de tissus enflammés inoculés avec le virus de la vaccine ou la préparation répond à la norme de qualité en effectuant la détermination ou l'évaluation selon l'une quelconque des revendications 1 à 5.

7. Procédé selon la revendication 6, dans lequel la préparation est une préparation injectable ou une préparation orale.
